# EUROPEAN PATENT APPLICATION

(11) **EP 4 491 121 A1**
(43) Date of publication of application: **15.01.2025**
(21) Application number: 24187726.5
(22) Date of filing: 10.07.2024
(51) Int. Cl.: A61B 5/346, A61B 5/35, A61B 5/352, A61B 5/00, A61N 1/37

(54) **PACEMAKER PULSE DETECTION SYSTEMS AND METHODS**

(30) Priority: 10.07.2023 US 202363512866 P
(71) Applicant: Analog Devices International Unlimited Company, Limerick (IE)
(72) Inventor: Munoz, Roberto, Limerick (IE)
(74) Representative: Yang, Shu

(57) **Abstract**

The present disclosure provides systems and methods for signal processing and detection for pacemaker pulses. Systems and methods can activate an analog-to-digital converter based on detecting a leading edge of a pacemaker pulse. Such use of the analog-to-digital converter may allow a system to operate at a lower power and with more specificity than certain other systems.

## Description

### BACKGROUND

### TECHNICAL FIELD

Embodiments of this disclosure relate to detecting pacemaker pulses.

### DESCRIPTION OF RELATED TECHNOLOGY

Pacemaker pulses can be detected from an electrocardiogram (ECG). Medical regulation specifies patient monitors (IEC60601-2-27), diagnostic ECGs (IEC60601-2-27) and ambulatory ECGs (IEC60601-2-47) such as Holter monitors, and ECG patches to monitor an ECG in the presence of pacemaker pulses. Such medical regulations can set technical specifications to detect pacemaker pulses and identify pacemaker pulses on the ECG record. ECG is typically a low amplitude (e.g., <10 millivolts (mV)) low bandwidth (e.g., < 150 hertz) signal, while pacemaker pulses are typically short (e.g., 0.1 millisecond (ms) to 2.0ms) pulses in the 2mV to 700mV amplitude range. Improved pacemaker pulse detection is desired for a variety of applications.

### SUMMARY

One aspect of the present disclosure is a method of pacemaker pulse detection and validation. The method includes detecting an edge of a pacemaker pulse in an analog domain with a first signal path; activating an analog-to-digital converter in a second signal path based on the detecting the edge of the pacemaker pulse; and validating the pacemaker pulse based on a digital output signal of the analog-to-digital converter.

The method can further include causing the analog-to-digital converter to stop digitizing an analog signal in response to detecting a second edge of the pacemaker pulse with the first signal path or after a time out. The validating can include performing a pulse shape check using a pulse validation circuit having an input coupled to an output the analog-to-digital converter. The method can include detecting a second edge of the pacemaker pulse in the analog domain with the first signal path, where the validating is based on an amount of time between the edge and the second edge passing a pulse width check. The validating can also be based on an output of a slew rate detector. The first signal path can provide an analog input signal that the analog-to-digital converter digitizes when activated. The method can include processing an analog signal with at least a high pass filter and an amplifier of the second signal path. An output of the amplifier can be coupled to an input of the analog-to-digital converter. The detecting can be performed by a detection circuit of the first signal path that performs differentiation, integration, and threshold comparison. The method can include detecting an edge of a second pulse in the analog domain with the first signal path; activating the analog-to-digital converter in the second signal path based on the detecting the edge of the second pulse; and determining that the second pulse is not a valid pacemaker pulse based on the digital output signal of the analog-to-digital converter.

Another aspect of the present disclosure is a system with pacemaker pulse detection. The system includes a first signal path including a detector circuit configured to detect a leading edge of a pacemaker pulse and a trailing edge of the pacemaker pulse; and a second signal path including an analog-to-digital converter that can activate based on the detector circuit detecting the leading edge and a pulse validation circuit. While activated, the analog-to-digital converter can digitize an analog signal comprising energy associated with the pacemaker pulse. The pulse validation circuit can have an input coupled to an output of the analog-to-digital converter, wherein the pulse validation circuit is configured to perform a pulse shape check

The analog-to-digital converter can stop digitizing based on the detector circuit detecting the trailing edge. The system can be configured to validate the pacemaker pulse based at least partly on an amount of time between the leading edge and the trailing edge. The analog-to-digital converter can receive the analog signal from the first signal path. The second signal path can include a high pass filter and an amplifier, and the analog-to-digital converter is configured to receive the analog signal from an output of the amplifier. The detector circuit can perform differentiation, integration, and threshold comparison.

Another aspect of the present disclosure is a system with pacemaker pulse detection. The system includes means for detecting edges of pacemaker pulses; and a analog - to-digital converter that can activate based on the means for detecting, wherein while activated the analog-to-digital converter digitizes an analog signal comprising energy associated with the pacemaker pulses; and means for validating the pacemaker pulse.

The means for validating can validate the pacemaker pulse based at least partly on an amount of time between the leading edge of the edges and the trailing edge of the edges. The means for validating can validate the pacemaker pulse based on a pulse shape check. The means for detecting detects rising edges and trailing edges of the pacemaker pulse. The means for detecting can perform differentiation, integration, and threshold comparison.

### BRIEF DESCRIPTION OF THE DRAWINGS

Embodiments of this disclosure will be described, by way of non-limiting example, with reference to the accompanying drawings.
Figure 1 is a schematic block diagram of a pacemaker pulse detection system according to an embodiment.
Figure 2 is a schematic block diagram of another pacemaker pulse detection system according to an embodiment.
Figure 3 is a schematic block diagram of another pacemaker pulse detection system according to an embodiment.
Figure 4 illustrates waveforms for edge detection and pulse validation for a valid pacemaker pulse.
Figure 5 illustrates a waveform for edge detection of an electromagnetic interference (EMI) and the resulting invalid pulse shape validation.
Figure 6 illustrates a waveform for edge detection of EMI and the resulting invalid pulse shape validation.
Figure 7 illustrates a waveform for edge detection output of a bandpass filtered noise input and the resulting invalid pulse shape validation.

### DETAILED DESCRIPTION OF CERTAIN EMBODIMENTS

The following detailed description of certain embodiments presents various descriptions of specific embodiments. However, the innovations described herein can be embodied in a multitude of different ways, for example, as defined and covered by the claims. In this description, reference is made to the drawings where like reference numerals can indicate identical or functionally similar elements. It will be understood that elements illustrated in the figures are not necessarily drawn to scale. Moreover, it will be understood that certain embodiments can include more elements than illustrated in a drawing and/or a subset of the illustrated elements. Further, some embodiments can incorporate any suitable combination of features from two or more drawings.

As discussed above, medical regulations involve patient monitors (IEC60601-2-27), diagnostic electrocardiograms (ECGs) (IEC60601-2-27) and ambulatory ECGs (IEC60601-2-47) such as Holter monitors, and ECG patches to monitor ECG in the presence of pacemaker pulses. These medical regulations can set technical specifications to detect pacemaker pulses and identify them on the ECG record.

An ECG is a relatively low amplitude (e.g., <10 mV) low bandwidth (e.g., < 150 Hz) signal, while pacemaker pulses are relatively short (e.g., 0.1 ms to 2.0 ms) pulses in the 2m V to 700 mV amplitude range.

Pacemaker pulses can be detected in the analog domain by monitoring energy (edges) in the 5 kilohertz (kHz) to 10 kHz range and setting a threshold. Such pacemaker pulse detection is a relatively low power approach. This approach can provide high sensitivity, but less specificity than may be desired. This can result in a relatively high number of false positives in noisy environments, environments with a high level on interference, or environments where medical equipment also connected to the same patient may create artifacts incorrectly detected by the ECG monitor as a pacemaker pulse.

Pacemaker pulses can also be detected by digitizing an incoming signal at relatively high data rate (e.g., > 40 kilo samples per second (kSPS)) to capture relatively fast edges and relatively short pulses. This can be a higher power approach than analog domain pacemaker pulse detection. Digitizing the incoming signal can involve a medium-resolution, fast analog-to-digital converter (ADC) that is consistently on, which can consume significant power. At the same time, digitizing an incoming signal to detect a pacemaker pulse can increase specificity relative to analog domain pacemaker pulse detection. Digital pacemaker pulse detection can also cause a manufacturer of the medical device to develop and validate a method for pacemaker pulse detection.

When a pacemaker fires a pulse its energy typically creates an artifact in the ECG, which is blanked by the ECG software (e.g., for a few tens or hundreds of milliseconds) once that pacemaker pulse is detected. If pulses are wrongly detected (i.e., false positives), the ECG is at risk of being fully blanked or there is a risk that portions of the ECG can repeatedly be blanked with a consequent loss of significant information for doctors and nurses. Accordingly, having a high specificity solution can be desirable.

It may be desirable to monitor patients for longer time. Patient monitors are evolving to a wearable and battery powered format where small size and low power consumption can be desirable. Reliable, high specificity pacemaker pulse detection tends to be a high power solution, which can be problematic for wearable applications. A high specificity and low power solution for pacemaker pulse detection is desired to enable next generation high acuity patient monitors.

Aspects of this disclosure relate to low power and high specificity pacemaker pulse detection. A low power path can control the duty cycling of an ADC that enables high specificity pacemaker pulse detection. A first signal path can be a low power signal path that includes a detector circuit configured to detect a first edge (e.g., a leading edge, also referred to herein as a rising edge) and a second edge (e.g., a trailing edge) of a pacemaker pulse in an analog domain, where the second edge has an opposite polarity than the first edge. A second signal path can be a high specificity path that includes an ADC to digitize an analog signal comprising high frequency energy associated with a pacemaker pulse. The ADC can be activated based on the detector circuit detecting the first edge of a pacemaker pulse. The ADC can be deactivated in response to the detector circuit detecting the second edge of the pacemaker pulse. The ADC can be deactivated after a timeout period if a trailing edge has not been detected within a threshold amount of time after the leading edge, where the threshold amount of time can be 2 ms or less. Accordingly, the first signal path can control the duty cycle of the ADC of the second signal path to maintain low power consumption while enabling high specificity pacemaker pulse detection. The second signal path can process a digital output of the ADC to validate that the edges detected by the first signal path indeed belonged to a valid pacemaker pulse. Low power and high specificity pacemaker pulse detection disclosed herein can be particularly advantageous in wearable battery powered next generation patient monitors, among other applications.

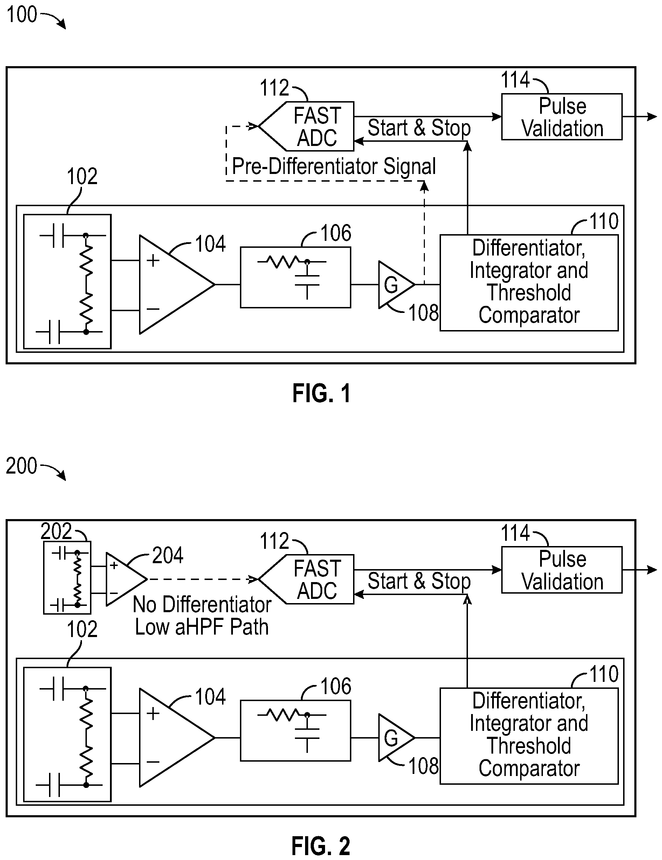

Pacemaker pulse detection systems with a plurality of signal paths for achieving high sensitivity and high specificity will be discussed with reference to Figures 1 to 3. These pacemaker pulse detection systems include a first signal path in an analog domain that can dynamically start and stop an ADC in a second signal path. The first signal path is low power and high sensitivity. The second signal path provides high specificity to validate pacemaker pulse detection in the first signal path. Such validation can reduce false positive pacemaker pulse detections. At the same time, a dual signal path solution can be low power due to the ADC and downstream circuitry only being on a relatively small percentage of time. The pacemaker pulse validation of the second signal path can reduce a burden on a microcontroller unit (MCU) from handling false positive pacemaker pulse detections. The second signal path including the ADC enables pulse validation techniques for difficult corners cases and/or on the fly learning for improved configuration and on the field upgrades for more reliable detection. The pacemaker pulse detection systems of Figures 1 to 3 provide a desirable tradeoff between low power and high specificity. This tradeoff can be particularly advantageous for wearables.

Figure 1 is a schematic block diagram of a pacemaker pulse detection system 100 according to an embodiment. The pacemaker pulse detection system 100 includes a first signal path and a second signal path. The first signal path includes a high pass filter 102, a first amplifier 104, a low pass filter 106, a second amplifier 108, and a detection circuit 110. The second signal path includes an ADC 112 and a pulse validation circuit 114. The first signal path and/or the second signal path can include more or fewer elements than illustrated as suitable. In certain applications, the first signal path and components of the second signal path can be implemented on a plurality of integrated circuits. According to some other applications, the first signal path and the second signal path can be implemented on a single integrated circuit.

The first signal path can always be on during operation of the pacemaker pulse detection system 100. The first signal path can detect edges of a pacemaker pulse. The first signal path detecting an edge of the pacemaker pulse can trigger the ADC 112 of the second signal path to activate or to stop digitizing an analog input signal.

An analog ECG signal can be received by the first signal path. The high pass filter 102 can filter out the analog ECG signal while letting through the high frequency energy associated with short pacemaker pulses. In certain applications, the high pass filter 102 can optionally be shared with a bioimpedance processing channel. The output of the high pass filter 102 can be provided to the first amplifier 104. The first amplifier 104 can be an input amplifier. The low pass filter 106 is coupled between the first amplifier 104 and the second amplifier 108 in the first signal path illustrated in Figure 1. The second amplifier 108 can be a programmable gain amplifier in certain applications. Overall, the high pass filter 102, the first amplifier 104, the low pass filter 106 and the second amplifier 108 can behave as a programmable gain band pass filter. The detection circuit 110 has an input coupled to an output of the second amplifier 108. The detection circuit 110 can include any suitable circuitry to implement a differentiator, an integrator, and a threshold comparator. The detection circuit 110 can perform differentiation, integration, and threshold comparison. The differentiator and integrator of the detection circuit 110 can behave as a more selective narrower bandpass filter. With such functionality, the detection circuit 110 can detect an edge of a pacemaker pulse. This edge detection can be eventually based on an amplitude voltage threshold. The edge detection can be implemented as a slew rate detector where both the slope and amplitude of the edge matter. The detection circuit 110 can detect a leading edge and a trailing edge of a pacemaker pulse, where either of these edges can have positive or negative polarity.

The ADC 112 can be activated in response to the detection circuit 110 detecting a leading edge of a pacemaker pulse. The ADC 112 can sample and digitize an analog input signal when in use. In the pacemaker pulse detection system 100, the analog input signal for the ADC 112 is an output signal of the second amplifier 108. In some other applications, the analog input signal for the ADC 112 can be tapped at another point along the signal chain, such as within the edge detection circuit 110. As illustrated in Figure 1, the analog input signal provided to the ADC 112 is not processed by the detection circuit 110. For example, the output signal of the second amplifier 108 is not processed by a differentiator (which can be equivalent to an aggressive high pass filter) because the analog input signal to the ADC 112 should have some amount of low frequency content so that the energy in between edges is maintained and can be used for pulse shape validation. The ADC 112 can be a relatively fast ADC. For example, the ADC 112 can sample at a rate of 40 kilo samples per second (kSPS) or greater. The ADC 112 can consume significant power when on, and the pacemaker pulse detection system 100 can maintain relatively low power by activating the ADC 112 for a relatively short amount of time. The ADC 112 can go into a low power mode in response to the detection circuit 110 detecting a trailing edge of the pacemaker pulse or a timeout event. In the low power mode of the ADC 112, the ADC 112 does not sample and digitize the analog input signal. The low power mode of the ADC 112 can be a standby mode. The ADC 112 is effectively deactivated in the low power mode. In some applications, during the inactivity time periods, the ADC 112 can sample at low sampling rates (e.g., low enough to keep this signal path low power) to track the baseline of the incoming signal.

The digital output signal from the ADC 112 can be processed in the digital domain. The pulse validation circuit 114 can perform one or more checks to validate the pacemaker pulse detection. The pacemaker pulse detection system 100 can perform checks to determine a valid pacemaker pulse detection. These checks can include an amplitude check, a pulse width check, and a pulse shape check. The detection circuit 110 can perform the amplitude check. The detection circuit 110 of the first signal path can detect edges of a pacemaker pulse based on amplitude detection, which can eventually be set by the edge's slope and pulse amplitude. Such amplitude detection can be based on a programmable threshold to perform the amplitude check. The pacemaker pulse detection system 100 (e.g., the detection circuit 110 or the pulse validation circuit 114) can perform the pulse width check. The pulse width check determines whether an amount of time between opposite edges of a pacemaker pulse is within a programable time window (e.g., typically between 100 microseconds (µs) to 2 ms). This can ensure that the pacemaker pulse has a proper pulse width. The pulse validation circuit 114 can perform a pulse shape check. The pulse validation circuit 114 can validate the shape of the pulse to boost the specificity of pacemaker pulse detection. The pulse validation circuit 114 can provide an output signal to a first-in first-out (FIFO) buffer where the pulse shape validation is performed. The pulse validation circuit 114 can provide an output signal to an MCU for the MCU to run the pulse validation on these heavily duty-cycled ADC samples.

In the pacemaker pulse detection system 100, part of the first signal path can be used to process an analog signal that is input to the ADC 112. Setting one or more parameters (e.g., corner frequency) of the high pass filter 102 can be significant in the pacemaker pulse detection system 100. The high pass filter 102 of Figure 1 can have a corner frequency that is sufficiently high to enable accurate edge detection by the first signal path (in combination with the differentiator in the detection circuit 110) and also sufficiently low to enable accurate pulse validation by the second signal path.

Figure 2 is a schematic block diagram of a pacemaker pulse detection system 200 according to an embodiment. The pacemaker pulse detection system 200 is like the pacemaker pulse detection system 100, except that (1) the second signal path additionally includes a dedicated high pass filter 202 and amplifier 204 and (2) the ADC 112 receives an analog input signal from the amplifier 204 instead of from the first signal path. By having a separate high pass filter 202 in the second signal path, the high pass filter 102 of the first signal path can have a more aggressive corner frequency for edge detection. At the same time, the high pass filter 202 of the second signal path can be set with a lower corner frequency for pulse validation in the second signal path. Accordingly, an analog signal comprising an ECG component and pacemaker pulse energy can be processed differently in the first signal path than in the second signal path in the pacemaker pulse detection system 200. In the pacemaker pulse detection system 200, the ADC 112 receives an analog signal that is processed in the second signal path. As illustrated in Figure 2, the ADC 112 receives the analog signal via the amplifier 204.

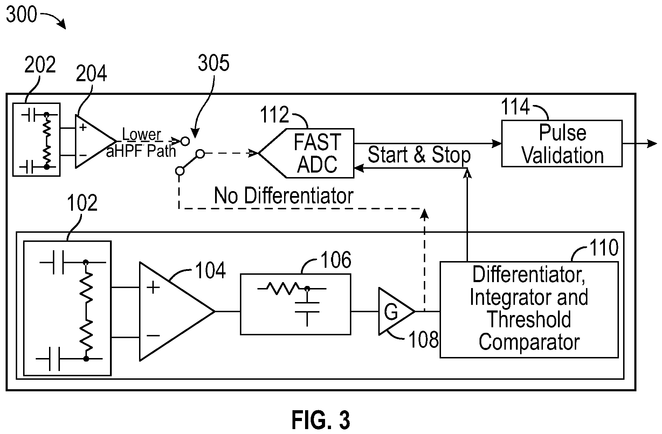

Figure 3 is a schematic block diagram of a pacemaker pulse detection system 300 according to an embodiment. The pacemaker pulse detection system 300 can operate in a first state (e.g., in accordance with the pacemaker pulse detection system 100) and operate in a second state (e.g., in accordance with the pacemaker pulse detection system 200). The pacemaker pulse detection system 300 includes a switch 305 that can selectively electrically couple either the amplifier 108 of the first signal path or the amplifier 204 of the second signal path to an input to the ADC 112. The pacemaker pulse detection system 300 can be programmed to operate in the first state or the second state by controlling the switch 305.

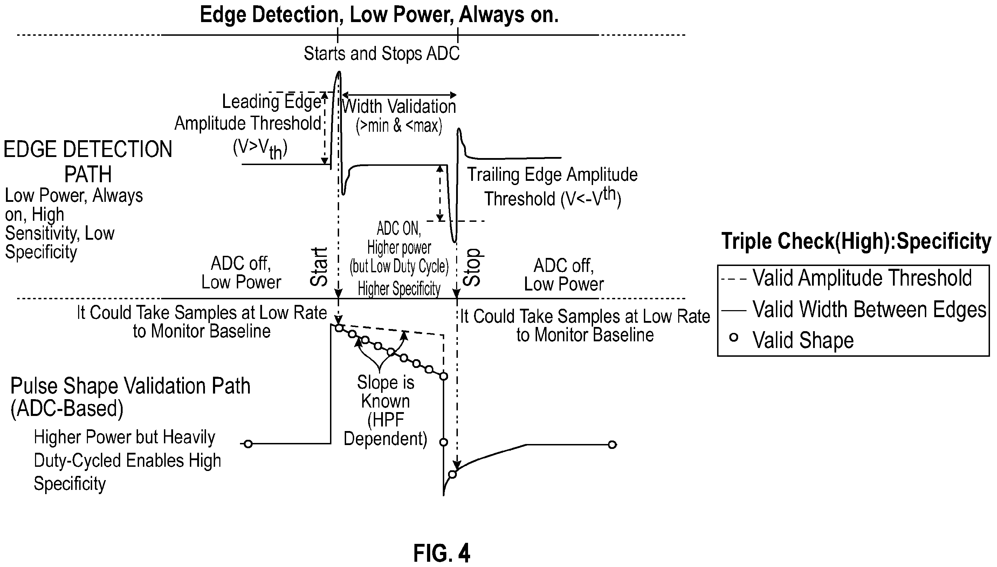

Figure 4 illustrates waveforms for edge detection and pulse validation for a valid pacemaker pulse. The edge detection waveform is effectively bandpass filtered by the first signal path. The detection circuit 110 can detect a leading edge and a trailing edge based on the amplitude of the edge detection waveform satisfying a threshold. A pulse width check on the edge detection waveform can check that the amount of time between the leading edge and the training edge is within a valid time window. The pulse validation circuit 114 can process the output signal from the ADC 112 to perform a pulse shape check. The relatively slow decay of the pulse shape validation waveform is high pass filter dependent, and therefore known within some tolerance. The pulse shape validation waveform of Figure 4 can pass the pulse shape check because it has a valid shape.

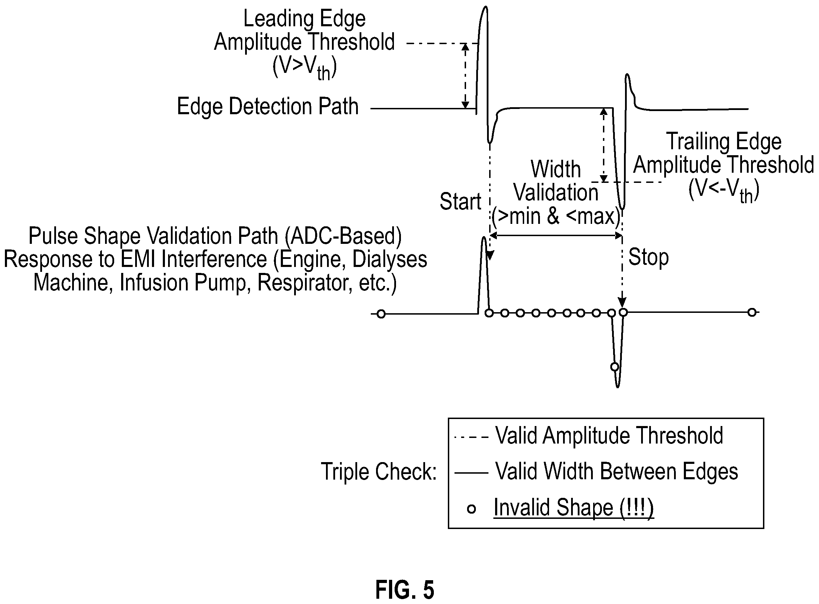

Figure 5 illustrates waveforms resulting from an electromagnetic interference (EMI) input. The EMI interference can be caused by a nearby engine, motor, dialysis machine, pump, etc. While the EMI waveform can pass an amplitude check and a pulse width check, the EMI waveform fails the pulse shape check. For example, the pulse validation circuit 114 can process the output signal from the ADC 112 to perform a pulse shape check to determine that the EMI waveform of Figure 5 is not a valid pacemaker pulse. The EMI waveform is an invalid shape because there is no energy between a leading edge and a trailing edge. Accordingly, pacemaker pulse detection systems disclosed herein can determine that the EMI waveform is not a valid pacemaker pulse.

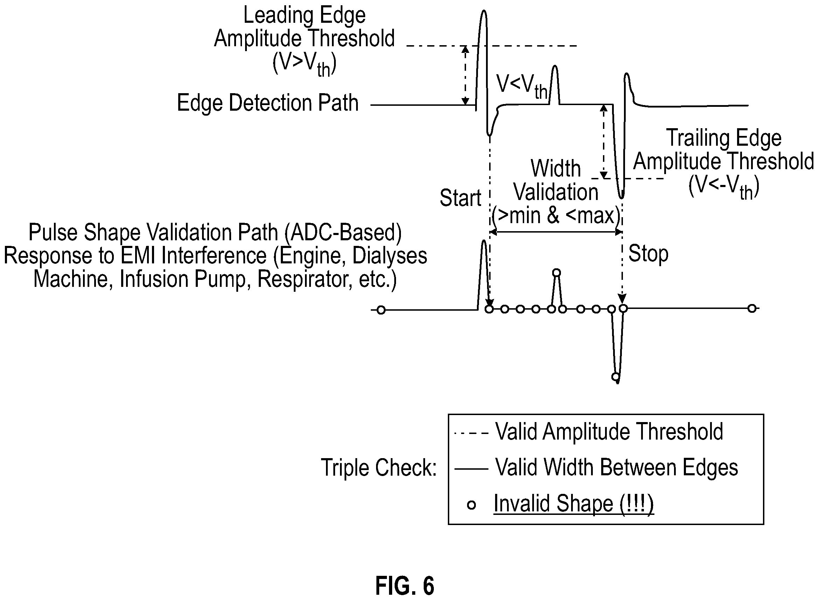

Figure 6 illustrates waveforms not corresponding to a pacemaker pulse. The waveform not corresponding to a valid pacemaker pulse can correspond to multiple EMIs. In Figure 6, the illustrated waveforms include a waveform for edge detection of EMI and the resulting invalid pulse shape validation. While the waveforms not corresponding to the pacemaker pulse can pass an amplitude check and a pulse width check, the waveform for pulse shape validation fails the pulse shape check. Accordingly, pacemaker pulse detection systems disclosed herein can determine that this waveform is not a valid pacemaker pulse. For example, the pulse validation circuit 114 can process the pulse shape validation path waveform of Figure 6 to determine that it is not a valid pacemaker pulse.

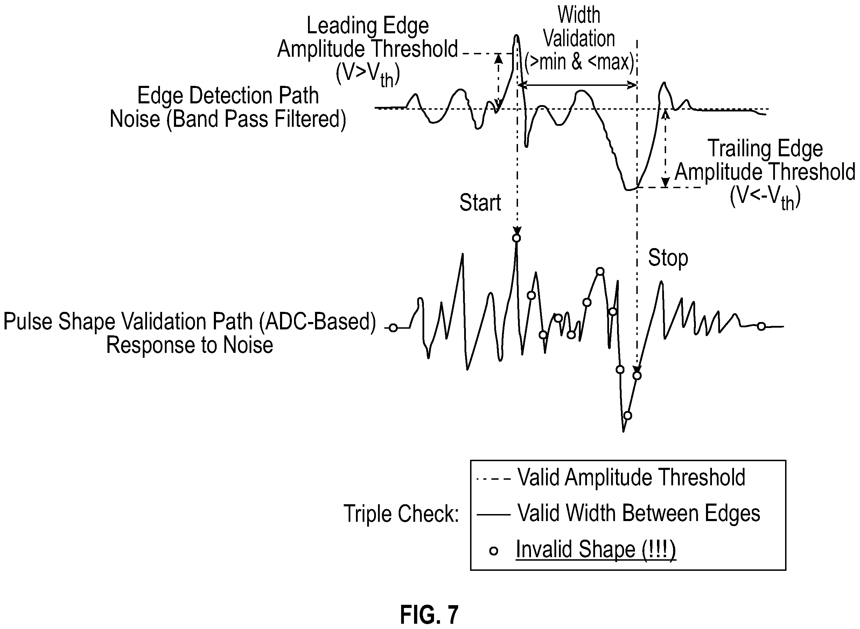

Figure 7 illustrates waveforms resulting from a noise input. The noise waveform can pass an amplitude check and a pulse width check. However, the noise waveform has an invalid pulse shape. For example, there is a lack of uniformity in a voltage drop expected for a valid pacemaker pulse between a leading edge and a trailing edge. The noise waveform should fail the pulse shape check. Accordingly, pacemaker pulse detection systems disclosed herein can determine that the noise waveform is not a valid pacemaker pulse. For example, the pulse validation circuit 114 can process the pulse shape validation path waveform of Figure 7 to determine that it is not a valid pacemaker pulse.

The pacemaker pulse detection systems disclosed herein can be implemented in a variety of different systems. Some such systems include wearable systems. In certain applications, at least part of a pacemaker pulse detection system disclosed herein can be implemented in a biopotential and bioimpedance analog front-end solution for wearable applications. Such a system can include one or more biopotential channels providing one or more of ECG waveforms, heart rate, and pacemaker pulse detection, and a bioimpedance channel capable of measuring respiration. Such a system can include one or more instances of the pacemaker pulse detection solution disclosed herein.

The pacemaker pulse detection systems disclosed herein can be in communication with one or more ECG sensors. The one or more ECG sensors can provide an input signal to the pacemaker detection system. The pacemaker detection system can have dedicated inputs different (although related) to the inputs of the ECG sensor.

In the embodiments described above, apparatus, systems, and methods for pacemaker pulse detection are described in connection with particular embodiments. It will be understood, however, that the principles and advantages of the embodiments can be used for any other systems, apparatus, or methods with a need for pacemaker pulse detection.

Unless the context clearly requires otherwise, throughout the description and the claims, the words "comprise," "comprising," "include," "including," and the like are to be construed in an inclusive sense, as opposed to an exclusive or exhaustive sense; that is to say, in the sense of "including, but not limited to." Additionally, the words "herein," "above," "below," and words of similar import, when used in this application, shall refer to this application as a whole and not to any particular portions of this application. Where the context permits, words in the Detailed Description using the singular or plural number may also include the plural or singular number, respectively. The words "or" in reference to a list of two or more items, is intended to cover all of the following interpretations of the word: any of the items in the list, all of the items in the list, and any combination of the items in the list. All numerical values provided herein are intended to include similar values within a measurement error.

Moreover, conditional language used herein, such as, among others, "can," "could," "might," "may," "e.g.," "for example," "such as" and the like, unless specifically stated otherwise, or otherwise understood within the context as used, is generally intended to convey that certain embodiments include, while other embodiments do not include, certain features, elements and/or states.

The teachings provided herein can be applied to other systems, not necessarily the systems described above. The elements and acts of the various embodiments described above can be combined to provide further embodiments. The acts of the methods discussed herein can be performed in any order as appropriate. Moreover, the acts of the methods discussed herein can be performed serially or in parallel, as appropriate.

While certain embodiments have been described, these embodiments have been presented by way of example only, and are not intended to limit the scope of the disclosure. Indeed, the novel methods and systems described herein may be embodied in a variety of other forms. Furthermore, various omissions, substitutions and changes in the form of the methods and systems described herein may be made without departing from the spirit of the disclosure. For example, while the disclosed embodiments are presented in given arrangements, alternative embodiments may perform similar functionalities with different components and/or circuit topologies, and some elements may be deleted, moved, added, subdivided, combined, and/or modified. Each of these elements may be implemented in a variety of different ways as suitable. Any suitable combination of the elements and acts of the various embodiments described above can be combined to provide further embodiments. The accompanying claims and their equivalents are intended to cover such forms or modifications as would fall within the scope and spirit of the disclosure. Accordingly, the scope of the present inventions is defined by reference to the claims.

### Numbered aspects

By way of non-limiting example, some aspects of the disclosure are set out in the following numbered clauses.
Numbered Clause 1. A method of pacemaker pulse detection and validation, the method comprising:
   detecting an edge of a pacemaker pulse in an analog domain with a first signal path;
   activating an analog-to-digital converter in a second signal path based on the detecting the edge of the pacemaker pulse; and
   validating the pacemaker pulse based on a digital output signal of the analog to-digital converter.
Numbered Clause 2. The method of Numbered Clause 1, further comprising causing the analog to digital converter to stop digitizing an analog signal in response to detecting a second edge of the pacemaker pulse with the first signal path or after a time out.
Numbered Clause 3. The method of Numbered Clause 1 or 2, wherein the validating comprises performing a pulse shape check using a pulse validation circuit having an input coupled to an output the analog-to-digital converter.
Numbered Clause 4. The method of any preceding Numbered Clause 1, further comprising detecting a second edge of the pacemaker pulse in the analog domain with the first signal path, wherein the validating is based on an amount of time between the edge and the second edge passing a pulse width check.
Numbered Clause 5. The method of Numbered Clause 4, wherein the validating is also based on an output of a slew rate detector.
Numbered Clause 6. The method of any preceding Numbered Clause, wherein the first signal path provides an analog input signal that the analog-to-digital converter digitizes when activated.
Numbered Clause 7. The method of any preceding Numbered Clause, further comprising processing an analog signal with at least a high pass filter and an amplifier of the second signal path, wherein an output of the amplifier is coupled to an input of the analog-to-digital converter.
Numbered Clause 8. The method of any preceding Numbered Clause, wherein the detecting is performed by a detection circuit of the first signal path that performs differentiation, integration, and threshold comparison.
Numbered Clause 9. The method of any preceding Numbered Clause, further comprising:
   detecting an edge of a second pulse in the analog domain with the first signal path;
   activating the analog-to-digital converter in the second signal path based on the detecting the edge of the second pulse; and
   determining that the second pulse is not a valid pacemaker pulse based on the digital output signal of the analog to-digital converter.
Numbered Clause 10. A system with pacemaker pulse detection, the system comprising:
   a first signal path comprising a detector circuit configured to detect a leading edge of a pacemaker pulse and a trailing edge of the pacemaker pulse; and
   a second signal path comprising:
      an analog-to-digital converter configured to activate based on the detector circuit detecting the leading edge, wherein while activated the analog-to-digital converter is configured to digitize an analog signal comprising energy associated with the pacemaker pulse; and
      a pulse validation circuit having an input coupled to an output of the analog to digital converter, wherein the pulse validation circuit is configured to perform a pulse shape check.
Numbered Clause 11. The system of Numbered Clause 10, wherein the analog-to-digital converter is configured to stop digitizing based on the detector circuit detecting the trailing edge.
Numbered Clause 12. The system of Numbered Clause 10 or 11, wherein the system is configured to validate the pacemaker pulse based at least partly on an amount of time between the leading edge and the trailing edge.
Numbered Clause 13. The system of any of Numbered Clauses 10 to 12, wherein the analog-to-digital converter is configured to receive the analog signal from the first signal path.
Numbered Clause 14. The system of any of Numbered Clauses 10 to 13, wherein the second signal path further comprises a high pass filter and an amplifier, and the analog-to-digital converter is configured to receive the analog signal from an output of the amplifier.
Numbered Clause 15. The system of any of Numbered Clauses 10 to 14, wherein the detector circuit is configured to perform differentiation, integration, and threshold comparison.
Numbered Clause 16. A system with pacemaker pulse detection, the system comprising:
   means for detecting edges of pacemaker pulses;
   an analog-to-digital converter configured to activate based on a signal from the means for detecting, wherein while activated the analog-to-digital converter digitizes an analog signal comprising energy associated with the pacemaker pulses; and
   means for validating the pacemaker pulse.
Numbered Clause 17. The system of Numbered Clause 16, wherein the means for validating is configured to validate the pacemaker pulse based at least partly on an amount of time between a leading edge of the edges and a trailing edge of the edges.
Numbered Clause 18. The system of Numbered Clause 16 or 17, wherein the means for validating is configured to validate the pacemaker pulse based on a pulse shape check.
Numbered Clause 19. The system of any of Numbered Clauses 16 to 18, wherein the means for detecting detects rising edges and trailing edges of the pacemaker pulses.
Numbered Clause 20. The system of any of Numbered Clauses 16 to 19, wherein the means for detecting is configured to perform differentiation, integration, and threshold comparison.

## Claims

1. A method of pacemaker pulse detection and validation, the method comprising:
detecting an edge of a pacemaker pulse in an analog domain with a first signal path;
activating an analog-to-digital converter in a second signal path based on the detecting the edge of the pacemaker pulse; and
validating the pacemaker pulse based on a digital output signal of the analog-to-digital converter.

2. The method of Claim 1, further comprising causing the analog-to-digital converter to stop digitizing an analog signal in response to detecting a second edge of the pacemaker pulse with the first signal path or after a time out.

3. The method of Claim 1 or 2, wherein the validating comprises performing a pulse shape check using a pulse validation circuit having an input coupled to an output the analog-to-digital converter.

4. The method of any preceding Claim, further comprising detecting a second edge of the pacemaker pulse in the analog domain with the first signal path, wherein the validating is based on an amount of time between the edge and the second edge passing a pulse width check.

5. The method of Claim 4, wherein the validating is also based on an output of a slew rate detector.

6. The method of any preceding Claim, wherein the first signal path provides an analog input signal that the analog-to-digital converter digitizes when activated.

7. The method of any preceding Claim, further comprising processing an analog signal with at least a high pass filter and an amplifier of the second signal path, wherein an output of the amplifier is coupled to an input of the analog-to-digital converter.

8. The method of any preceding Claim, wherein the detecting is performed by a detection circuit of the first signal path that performs differentiation, integration, and threshold comparison.

9. The method of any preceding Claim, further comprising:
detecting an edge of a second pulse in the analog domain with the first signal path;
activating the analog-to-digital converter in the second signal path based on the detecting the edge of the second pulse; and
determining that the second pulse is not a valid pacemaker pulse based on the digital output signal of the analog-to-digital converter.

10. A system with pacemaker pulse detection, the system comprising:
a first signal path comprising a detector circuit configured to detect a leading edge of a pacemaker pulse and a trailing edge of the pacemaker pulse; and
a second signal path comprising:
an analog-to-digital converter configured to activate based on the detector circuit detecting the leading edge, wherein while activated the analog-to-digital converter is configured to digitize an analog signal comprising energy associated with the pacemaker pulse; and
a pulse validation circuit having an input coupled to an output of the analog-to-digital converter, wherein the pulse validation circuit is configured to perform a pulse shape check.

11. The system of Claim 10, wherein the analog-to-digital converter is configured to stop digitizing based on the detector circuit detecting the trailing edge.

12. The system of Claim 10 or 11, wherein the system is configured to validate the pacemaker pulse based at least partly on an amount of time between the leading edge and the trailing edge.

13. The system of any of Claims 10 to 12, wherein the analog-to-digital converter is configured to receive the analog signal from the first signal path.

14. The system of any of Claims 10 to 13, wherein the second signal path further comprises a high pass filter and an amplifier, and the analog-to-digital converter is configured to receive the analog signal from an output of the amplifier.

15. The system of any of Claims 10 to 14, wherein the detector circuit is configured to perform differentiation, integration, and threshold comparison.
